# EUROPEAN PATENT SPECIFICATION

(11) **EP 3 924 104 B1**
(45) Date of publication and mention of the grant of the patent: **28.01.2026**
(21) Application number: 20708533.3
(22) Date of filing: 12.02.2020
(51) Int. Cl.: B01J 20/285, B01J 41/05, B01J 41/07

(54) **A METHOD OF DETECTING CARBON DIOXIDE IN A GASEOUS SAMPLE AND APPARATUS FOR PERFORMING THE METHOD**
VERFAHREN ZUM NACHWEIS VON KOHLENDIOXID IN EINER GASFÖRMIGEN PROBE UND VORRICHTUNG ZUR DURCHFÜHRUNG DES VERFAHRENS
PROCÉDÉ DE DÉTECTION DE DIOXYDE DE CARBONE DANS UN ÉCHANTILLON GAZEUX ET APPAREIL POUR LA MISE EN OEUVRE DE CE PROCÉDÉ

(30) Priority: 12.02.2019 FI 20195103
(43) Date of publication of application: 22.12.2021
(73) Proprietor: Teknologian tutkimuskeskus VTT Oy, 02150 Espoo (FI)
(72) Inventor: GENOUD, Guillaume, 02044 VIT (FI); REINIKAINEN, Matti, 02044 VIT (FI)
(74) Representative: Laine IP Oy
(86) International application number: PCT/FI2020/050085
(87) International publication number: WO 2020/165500

(56) References cited:
- US-A1- 2011 201 126
- US-A1- 2017 284 993
- W. RICHARD ALESI ET AL: "Evaluation of a Primary Amine-Functionalized Ion-Exchange Resin for CO 2 Capture", INDUSTRIAL & ENGINEERING CHEMISTRY RESEARCH, vol. 51, no. 19, 7 May 2012 (2012-05-07), pages 6907 - 6915, XP055581507, ISSN: 0888-5885, DOI: 10.1021/ie300452c
- GENOUD G ET AL: "Radiocarbon dioxide detection based on cavity ring-down spectroscopy and a quantum cascade laser", OPTICS LETTERS, OSA, vol. 40, no. 7, 1 April 2015 (2015-04-01), pages 1342 - 1345, XP001594705, DOI: 10.1364/OL.40.001342

## Description

### FIELD

The present invention relates to carbon dioxide isotopologue detection methods, and more particularly optical radiocarbon detection methods.

### BACKGROUND

Carbon has two stable isotopes and an unstable isotope, carbon-14 also called radiocarbon (C-14). It is present in trace amounts on Earth, with an abundance compared to the main carbon isotope (¹⁴C/¹²C) of 1.2 part per trillion (ppt). Radiocarbon is produced from nitrogen by thermal neutrons, either naturally in upper atmosphere or in anthropogenic nuclear reactions, e.g. nuclear power plants or past atmospheric nuclear weapon tests. It then enters the carbon cycle and is present in all modern carbon, while it has decayed to a negligible level in fossil carbon due to its half-life of 5730 years. It is therefore the ideal tracer for discriminating between emissions of fossil origin or biogenic origin, and has numerous applications. It is for instance used to monitor the biofraction in mixed fuels for carbon trade schemes, and to evaluate the contribution of fossil emissions to the global greenhouse gas emissions. C-14 is also commonly used in biomedicine to label organic compounds.

C-14 is also one of the main sources of radioactive gas emissions in nuclear facilities, and regulations require it to be monitored.

In nuclear facilities C-14 can be found in concentrations higher than its natural abundance, typically about 1 ppb to 1 ppm. All parts of nuclear power plants are potential sources for radiocarbon emissions in gaseous form, mostly in the form of carbon dioxide but also in other molecular forms such as methane. In waste repositories, for example, biodegradation of radioactive waste produces ¹⁴CO₂ emissions at levels in the range 10 ppb to 1 ppm. Such levels correspond to activity concentrations in the range 1 to 100 Bq/ml. Long-lived radioisotopes such as radiocarbon are particularly challenging to detect in the context of nuclear facilities.

Stable isotopes of CO₂, also called CO₂ isotopologues, are used as a tracer of the origin of emissions from different types of processes, such as photosynthesis, respiration, or combustion of fossil fuels. These emissions will have different isotopic signatures. Atmospheric CO₂ isotopic measurement is therefore an important tool in atmospheric research.

Carbon isotopes (C-13 and C-14) are regularly used to label molecules to follow biological complex processes in biomedical applications.

An accelerator mass spectrometer is the state-of-the-art instrument for radiocarbon detection, while liquid scintillation counting is also extensively used in particular in nuclear facilities. These methods have several drawbacks. They are mainly laboratory-based thus requiring off-site sample analysis, which is a disadvantage when large numbers of samples must be analysed or real-time on-line monitoring is needed.

Radiocarbon detection using laser spectroscopy has on-site on line measurement capabilities, and in the future it can benefit many applications in the fields of nuclear safety, biomedicine, and environmental monitoring. This optical technique relies on the detection of absorption lines of ¹⁴CO₂ by using mid-infrared laser spectroscopy.

For example I. Galli et al. describe the determination of radiocarbon by using saturated-absorption cavity ring-down spectroscopy in Optica 3 (2016) 385-388.

A. J. Fleisher et al. describe optical measurement of radiocarbon below unity fraction modern by linear absorption spectroscopy in The Journal of Physical Chemistry Letters 0, PMID: 28880564, 4550 (2017).

A. D. McCartt et al. describe measurements of carbon-14 with cavity ring-down spectroscopy in Nucl. Instr. Meth. Phys. Res. B 361, 277 (2015).

Stable isotopes can be detected using isotope ratio mass spectrometer, which is also a laboratory-based method. Optical methods for stable isotope detection are commercially available, but the size and cost of such equipment are large.

N₂O is present in trace amounts (about 330 ppb) in the atmosphere but it has strong absorption lines in the 4.0 to 4.5 microns wavelength region. In laser spectroscopy applications, these absorption lines can interfere with the measurement and thus reduce the sensitivity, in particular in applications that rely on radiocarbon detection in the form of carbon dioxide, because absorption lines in the same wavelength region are used for its detection. Strong N₂O absorption lines are present close to ¹⁴CO₂ absorption lines that are used for radiocarbon detection.

In order to determine the isotopic composition of gaseous emissions, it is sometime necessary to concentrate the targeted gas in order to achieve the highest sensitivity. This is particularly important for radiocarbon detection, as the natural abundance of C-14 is extremely small. For example, CO₂ concentration in air is only 400 ppm, so in order to achieve the highest sensitivity it is necessary to first extract CO₂ from air before further analysis.

Current methods for CO₂ extraction and radiocarbon detection require laboratory sample preparation and are time consuming, thus not suitable for on-line in-situ measurements.

Some methods to get pure CO₂ for further analysis are using molecular sieves to trap the CO₂. Even though these methods are very effective, the trapping and release times are very long, thus leading to very low acquisition rates and making such techniques unsuitable for in-situ on-line measurements.

Trapping methods based on the freeze-and-release principle and employing a cryogenic trap are extensively used in the laboratory, but such methods require use of liquid nitrogen, which is not compatible with field measurements. Portable cryogenic coolers can also be used, but they are very expensive and relatively complex to operate. Most importantly, such methods also trap N₂O, which interferes with a spectroscopic measurement.

Anion exchange resins that are capable of adsorbing CO₂ have been described previously.

A. Yoshida, et. al describe the use of macroreticular resins in the article "Adsorption of CO2 on composites of strong and weak basic anion exchange resin and chitosan", J. Chem. Eng. of Japan 35 (2002) 32-39.

US 2007/0217982 A1 describes an apparatus for removal of CO₂ from the atmosphere comprising an anion exchange material formed in a matrix exposed to a flow of the air.

US 2017/284993 A1 discloses an apparatus for analyzing isotope ratios in fractions of samples such as petroleum source and reservoir rocks, kerogen and bitumen isolates, and drill cuttings, which apparatus has a dynamically heated chamber, a reactor and an isotope ratio spectrometer; a selective gas trap provided in the gas flow path, and is configured to selectively and reversibly trap one or more gases present in the gas flow. US 2017/284993 discloses that selective gas trap may comprise a sorbent material, selected to specifically trap the analyte gas, whilst allowing other gases to pass through the trap. The sorbent material may adsorb the analyte gas. The sorbent may comprise a metal oxide, a polymer or a molecular sieve.

There is a need for developing a cheap and simple method of selectively concentrating carbon dioxide before analysis of carbon isotopes in the carbon dioxide.

There is a need for developing a sensitive method for the detection of radiocarbon in various molecular forms, particularly ¹⁴CO₂ and ¹⁴CH₄.

There is a further need for providing an online and onsite method for monitoring radiocarbon.

The embodiments of the present invention are intended to overcome at least some of the above discussed disadvantages and restrictions of the prior art.

### SUMMARY OF THE INVENTION

The invention is defined by the features of the independent claims. Some specific embodiments are defined in the dependent claims.

According to a first aspect of the present invention, there is provided a method of detecting carbon dioxide in a gaseous sample, the method comprising: flowing the gaseous sample through an anion exchange resin that is capable of selectively adsorbing CO₂ present in the gaseous sample; releasing the adsorbed CO₂ from the resin by heating the resin to a temperature in the range 80 to 250 °C to obtain a concentrated gaseous sample; determining the amount of an isotopic form of CO₂ in the concentrated gaseous sample by infrared absorption spectroscopy, wherein the anion exchange resin features primary, secondary, and/or tertiary amino groups.

Various embodiments of the first aspect may comprise at least one feature from the following bulleted list:
- The isotopic form is ¹²CO₂ or ¹³CO₂ or ¹⁴CO₂ or any combination of them.
- The isotopic form of CO₂ is any isotopologue of CO₂ containing any isotope(s) of oxygen and any isotope of carbon.
- The anion exchange resin comprises crosslinked polymeric material.
- The resin is heated to a temperature in the range 100 to 200 °C, for example 150 to 200 °C.
- The resin is heated for a time period of 1 to 15 minutes, preferably for 10 minutes at maximum.
- The determining step comprises measuring an infrared absorption spectrum of the concentrated gaseous sample by using a cavity down-ring laser spectroscopy.
- The isotopic form is ¹⁴CO₂; the gaseous sample further comprises ¹⁴CH₄, and the method further comprises, before the determination step: catalytically oxidizing the ¹⁴CH₄ to ¹⁴CO₂ by a catalyst, whereby the ¹⁴CO₂ to be determined in the determination step also comprises ¹⁴CO₂ converted from the ¹⁴CH₄ present in the gaseous sample.
- The catalyst is a Pd catalyst, and the step of catalytically oxidizing the ¹⁴CH₄ to ¹⁴CO₂ comprises: heating the Pd catalyst to a temperature of at least 300 °C; bringing the gaseous sample into contact with the heated Pd catalyst; whereby the heated Pd catalyst catalyses oxidation of the ¹⁴CH₄ present in the gaseous sample to ¹⁴CO₂.
- The isotopic form is ¹⁴CO₂ and the gaseous sample originates from a nuclear power plant.
- The gaseous sample is an atmospheric sample.
- The gaseous sample is/originates from biofuels, such as biodiesel or biogas.
- The gaseous sample is/originates from a biological sample, such as a breath sample, a blood sample or a plasma sample.

According to a second aspect of the present invention, there is provided an apparatus comprising in a cascade: first means for concentrating CO₂ present in a gaseous sample to obtain a concentrated gaseous sample; and second means for determining the amount of an isotopic form of CO₂ present in the concentrated gaseous sample by infrared absorption spectroscopy, wherein the first means comprises an anion exchange resin that is capable of selectively adsorbing CO₂ present in the gaseous sample, wherein the anion exchange resin features primary, secondary, and/or tertiary amino groups.

Various embodiments of the second aspect may comprise at least one feature from the following bulleted list:
- The isotopic form is ¹²CO₂ or ¹³CO₂ or ¹⁴CO₂ or any combination of them.
- The isotopic form of CO₂ is any isotopologue of CO₂ containing any isotope(s) of oxygen and any isotope of carbon.
- The isotopic form is ¹⁴CO₂; and the apparatus further comprises: upstream of said first means, further means for catalytically oxidizing ¹⁴CH₄ present in the gaseous sample, wherein said second means is adapted for determining the combined amount of ¹⁴CO₂ present in the gaseous sample and ¹⁴CO₂ converted from the ¹⁴CH₄ present in the gaseous sample by infrared absorption spectroscopy.
- The further means for catalytically oxidizing ¹⁴CH₄ present in the gaseous sample comprises a catalyst bed comprising a catalyst, preferably a Pd catalyst.
- The second means comprises a cavity down-ring laser spectrometer comprising a quantum cascade laser as an IR light source.

The present invention provides numerous advantages.

Some embodiments of the present method make it possible to simultaneously concentrate carbon dioxide from gaseous samples and to eliminate interference from N₂O for the purpose of spectroscopic determination of ¹⁴CO₂ and also other isotopic forms of carbon dioxide.

The present method makes it possible to concentrate carbon dioxide before analysis of carbon isotopes and possibly also oxygen isotopes in the carbon dioxide.

Conventional methods cannot differentiate between the different molecular forms of C-14, i.e. different compounds containing C-14. Some embodiments of the present method overcome this drawback.

Some embodiments of the present invention provide a sensitive spectroscopic method for detecting radiocarbon in gaseous samples. We have observed that laser spectroscopy can be successfully applied to the monitoring of radiocarbon in various molecular forms.

While the conventional method of liquid scintillation counting for radiocarbon detection relies on detecting emitted radiation, the present invention is based on detecting the underlying molecular species by spectroscopic means. Some embodiments of the present invention avoid any interference from other radioactive elements, such as tritium.

Some embodiments of the invention provide a much simpler and more affordable way of selectively trapping CO₂ for isotopic analysis using laser spectroscopy without trapping unwanted contaminants, most importantly N₂O.

In the present method, an anion exchange resin is used to selectively adsorb CO₂ while unwanted contaminants such as N₂O do not become adsorbed. This is especially important for laser spectroscopy applications related to CO₂ isotopes as even trace amounts of N₂O can interfere with the measurement.

In the case of stable CO₂ isotopes, CO₂ purification (concentration) by means of some embodiments of the present invention makes it possible to reduce the cost and size of present optical instruments.

### BRIEF DESCRIPTION OF THE DRAWINGS

FIGURE 1 illustrates schematically a laser spectroscopy apparatus, more specifically a cavity ring-down spectroscopy setup, in accordance with at least some embodiments of the present invention;
FIGURE 2 shows results from an experiment that studies desorption of CO₂ from a resin in accordance with an embodiment of the present invention;
FIGURE 3 shows results from an experiment that studies eventual adsorption of N₂O by an anion exchange resin in accordance with an embodiment of the present invention;
FIGURE 4A shows an absorption spectrum measured in a method according to an embodiment of the present invention, using an anion exchange resin for extracting CO₂;
FIGURE 4B shows a comparative absorption spectrum measured in a method that uses cryogenic trapping for extracting CO₂; and
FIGURE 5 illustrates an experimental set-up for fast sampling in accordance with an embodiment of the present invention.

### EMBODIMENTS

The present method uses an anion exchange resin to trap CO₂, wherein the anion exchange resin features primary, secondary, and/or tertiary amino groups. Only a small amount of CO₂ is required for a spectroscopic analysis, and accordingly a small amount of the resin is sufficient. Further, the trapping time is short. This type of resin allows efficient trapping and fast release of the trapped CO₂.

We have surprisingly observed that the resin does not adsorb N₂O, which is a critical condition for carrying out a spectroscopic analysis of carbon dioxide isotopes, particularly ¹⁴CO₂.

In the present context, "isotopologues" are molecules that differ only in their isotopic composition. The isotopologue of a chemical species has at least one atom with a different number of neutrons than the parent.

In the present context, the term "radiocarbon" refers to ¹⁴C, the radioactive isotope of carbon.

In the present context, the term "weakly basic" anion exchange resin refers to for example a resin that does not contain exchangeable ionic sites and functions as acid adsorber. The different types of ion-exchange resins differ mainly in their functional groups. Weakly basic resins typically feature primary, secondary, and/or tertiary amino groups, for example polyethylene amine.

Air samples usually contain trace amounts of N₂O, which has strong absorption lines close to the CO₂ absorption line in the mid-infrared wavelength range. In the case of detecting ¹²CO₂, such trace amounts would not pose any problem, because the levels of ¹²CO₂ in the air are in the range 400 ppm to a few %. For the purpose of monitoring ppt levels of ¹⁴CO₂, the interference from N₂O significantly decreases sensitivity.

The inventors have surprisingly observed that the interference arising from N₂O in laser spectroscopic radiocarbon detection methods can be successfully eliminated by using an anion-exchange resin for concentrating CO₂.

According to some embodiments of the present invention, any isotopologue of carbon dioxide can be detected, preferably selected from the unstable isotopologues of carbon dioxide, such as the unstable isotopologues containing at least one of the following: C-13, C-14, O-17, O-18.

According to some embodiments of the present invention, several isotopologues of carbon dioxide can be detected, preferably the stable carbon dioxide CO₂ in combination with any of the unstable isotopologues of carbon dioxide.

According to an embodiment, the isotopologue is ¹⁴C¹⁶O¹⁶O.

According to an embodiment, the isotopologue is ¹³C¹⁶O¹⁶O.

According to an embodiment, the isotopologue is ¹²C¹⁶O¹⁸O.

According to an embodiment, the isotopologue is ¹²C¹⁶O¹⁷O.

According to an embodiment, the isotopologue is ¹³C¹⁶O¹⁸O.

According to an embodiment, the isotopologue is ¹³C¹⁶O¹⁷O.

According to an embodiment, the isotopologue is formed by an unstable isotope of carbon (¹⁴C or ¹³C) and any isotope(s) of oxygen as any combination.

According to an embodiment, the isotopologue is formed by the stable isotope of carbon (¹²C) and any isotope(s) of oxygen as any combination.

In some embodiments of the present invention, the concentration of ¹⁴CO₂ in the sample can be increased from ppq or ppb levels to ppt or ppm levels.

While traditional radiation detectors rely on the detection of emitted radiation, the method presented here detects the molecules containing the radioisotope C-14 itself. The present method is based on optical methods for the detection of molecules containing radiocarbon.

Radiocarbon is a beta emitter. In the present invention, it is not necessary to chemically separate other beta emitters, such as tritium, beforehand, which is an advantage over traditional radiochemistry methods, such as liquid scintillation counting.

In some embodiments of the present invention, radiocarbon originally present in different molecular forms is detected in the form of carbon dioxide (¹⁴CO₂).

The invention provides several advantages in terms of size, price, and on-site measurement capabilities. The system presented here enables automated onsite and online monitoring of fugitive radiocarbon emissions in nuclear facilities.

In one embodiment, before trapping ¹⁴CO₂, ¹⁴CH₄ present in the sample is catalytically oxidized to CO₂ by a catalyst according to the following reaction:

CH₄ + O₂ → CO₂

The catalyst is preferably a Pd catalyst, for example an alumina supported Pd catalyst.

In one embodiment, the catalyst is a Pd catalyst comprising 2 to 3 wt-% Pd.

In some embodiments, the Pd catalyst is prepared by the method described in Fouladvand et al., "Methane Oxidation Over Pd Supported on Ceria-Alumina Under Rich/Lean Cycling Conditions", Topics in Catal. (2013) 56:410-415.

Other possible catalysts for catalysing oxidation of ¹⁴CH₄ are precious metals, such as platinum or palladium or rhodium.

During the catalytic oxidation of ¹⁴CH₄ by the catalyst, the temperature is preferably at least 285 °C, more preferably in the range 300 to 500 °C, most preferably in the range 300 to 350 °C.

### Anion exchange separation

The anion exchange resin features primary, secondary, and/or tertiary amino groups, e.g. polyethylene amine.

In one embodiment, the anion exchange resin is a crosslinked polystyrene based resin, preferably functionalized with amino groups.

In one embodiment, the anion exchange resin is a polystyrene polymer based resin, which is crosslinked via the use of divinylbenze, and is functionalized with primary amine groups, such as benzylamine. Such a resin can be produced by a phthalimide process, for example by a process that is commercially available from LANXESS Deutschland GmbH under the brand name LEWATIT^{®} VP 001065.

In one embodiment, LEWATIT^{®} VP OC 001065 resin is used. According to literature (Alesi & Kitchin, Ind. Eng. Chem. Res. 2012, 51, 6907-6915) the capture capacity of LEWATIT VP OC 001065 resin is remarkably high; 1.85 to 1.15 mol CO₂/kg in a packed bed reactor exposed to 10 vol-% of CO₂ at adsorption temperatures ranging from 30 to 70 °C.

In one embodiment, the anion exchange resin is a weakly basic purely gel-type resin.

The thermal stability of the resin must be high enough to facilitate fast regeneration. Therefore, the resin preferably comprises crosslinked polymeric material.

In one embodiment, the gaseous sample is flown through a column containing the resin, whereby the CO₂ present in the sample becomes adsorbed.

In preferred embodiments, to release the adsorbed CO₂, the resin is heated, preferably to a temperature in the range 100 to 250 °C, for example 150 to 200 °C. It is advantageous to keep the temperature below 250 °C, for example below 170 °C, so that nitrogen-containing functional groups in the resin do not decompose or react and produce interfering N₂O.

The duration of the heating step is preferably 1 to 15 minutes, more preferably 10 minutes at maximum. A short and fast heating is preferred so that nitrogen-containing functional groups in the resin do not decompose or react and produce interfering N₂O.

In some embodiments, multiple columns, at least two columns, can be arranged in parallel to enable continuous or at least faster sampling. One cycle of trapping a sample, heating the resin, cooling the resin and regenerating the resin typically takes about 30 minutes. By using parallel columns, sampling for example at 5-minute intervals becomes possible.

### Optical measurement

The optical detection is based on measuring infrared absorbance of the sample. The preferred wavenumber range is 2200 to 2250 cm⁻¹. The preferred absorption line of CO₂ for determining the amount of radiocarbon in the form of ¹⁴CO₂ is situated at 2209.1 cm⁻¹.

Preferably, the light source is a tunable laser, for example a quantum cascade laser, or an optical parametric oscillator.

In one embodiment, the optical detection method is a cavity ring-down spectroscopic method, and light is detected by an infrared photovoltaic detector at the output of the cavity.

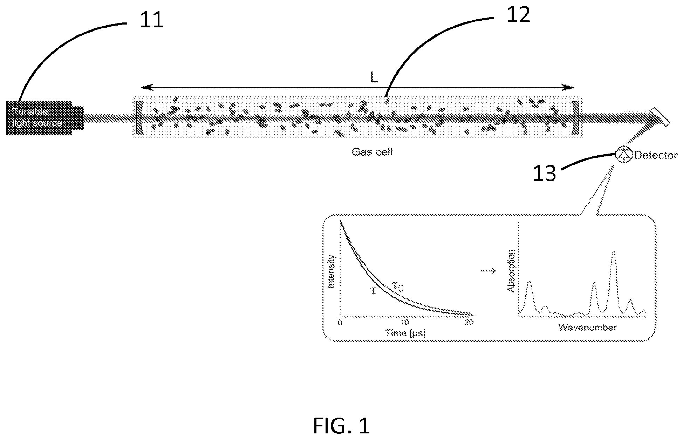

FIGURE 1 illustrates schematically a laser spectroscopy apparatus in accordance with at least some embodiments of the present invention. The apparatus comprises a tunable light source 11, a gas cell 12 in form of a cavity, and a detector 13 at the output of the gas cell. The length L of the gas cell is for example 40 cm. Absorption is measured as a function of wavenumber.

In some embodiments, the spectroscopic set-up described in the publication G. Genoud et al., "Radiocarbon dioxide detection based on cavity ring-down spectroscopy and a quantum cascade laser", Optics Letters 40 (2015) 1342-1345, and comprising a cavity down-ring spectrometer, a quantum cascade laser and an infrared photovoltaic detector is used.

### EXAMPLES

### Example 1: Adsorption and release of CO₂

Air samples are flown through the resin at room temperature. The CO₂ present in the sample becomes trapped. Then the resin is heated to a temperature in the range 150 to 200 °C, whereby pure CO₂ is released and can be lead to spectroscopic analysis. A sufficient amount of CO₂ can be trapped in about 5 to 10 minutes. The trapped CO₂ is almost instantly released when the resin reaches the required temperature, for example 150 °C.

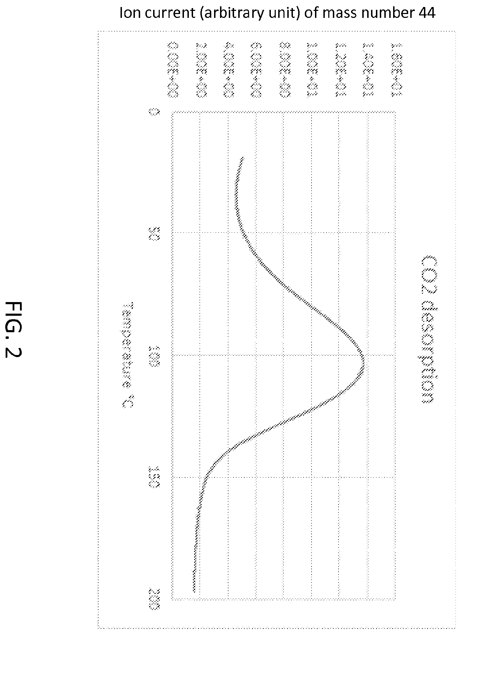

FIG. 2 shows results from an experiment in which we studied desorption of CO2 from a resin. 0.5 g of Lewatit VP OC 1065 resin was placed in a quartz tube. The resin was heated up to 200 °C in a He flow to clean its surface and then cooled down to 25°C. The effluent gas was analyzed using a quadrupole mass spectrometer (QMS). Carbon dioxide pulses of 1 ml were added to helium flow until the resin was saturated with CO₂. The size of the peak following each pulse in the QMS remained constant. The resin was heated in helium flow ramping the temperature at a rate of 30 °C/min while monitoring the desorption by means of QMS (ion current vs. temperature). The graph shows ion current (arbitrary unit) of mass number 44 as a function of temperature (°C).

### Example 2: Testing of N₂O adsorption

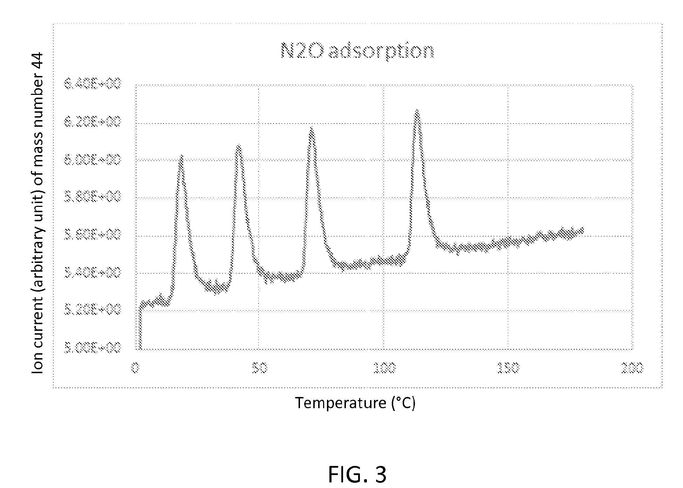

FIG. 3 shows results from an experiment in which we tested to what extent N₂O is adsorbed by the anion exchange resin in accordance of an embodiment of the present invention.

The setup and procedure was the same as in the case of FIG. 2 except that the resin was pulsed with a gas mixture containing 100 ppm of N₂O in nitrogen. The size of the peaks remained constant from the first pulse and no adsorption of N₂O could be detected. Also the desorption curve did not show any desorbing N₂O. The graph shows ion current (arbitrary unit) of mass number 44 as a function of temperature (°C).

### Example 3: Comparative experiments by using either an anion exchange resin or a cryogenic trap for the extraction of CO₂.

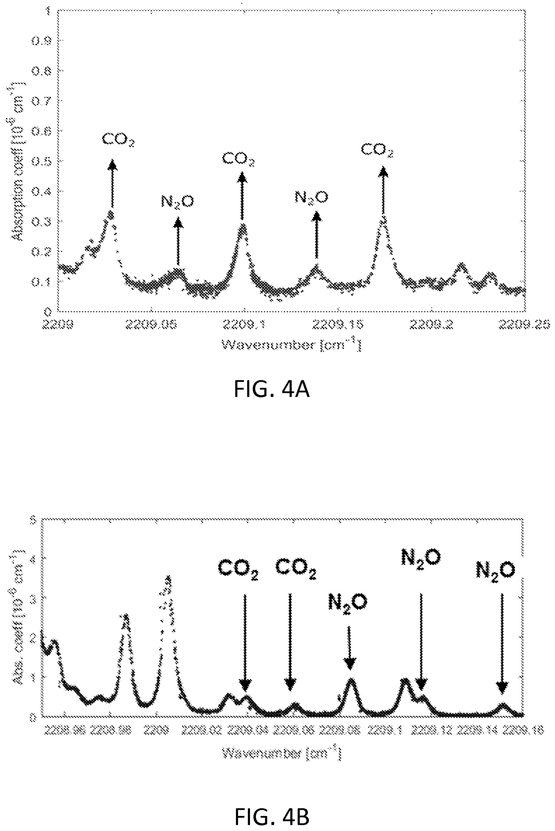

FIGURE 4A shows an absorption spectrum measured in a method according to an embodiment of the present invention, using an anion exchange resin for extracting CO₂. FIGURE 4B shows a comparative absorption spectrum measured in a method that uses cryogenic trapping for extracting CO₂. In both graphs, absorption coefficient is shown as a function of wavenumber. It was observed that the resin selectively adsorbs carbon dioxide without adsorbing N₂O. A small amount of N₂O may be released when the resin is heated to a high temperature; this phenomenon can be further reduced by minimizing the heating time. When the cryogenic trap was used, peaks originating from N₂O have a high intensity in relation to CO₂ peaks, which decreases the accuracy of CO₂ determination.

### Example 4: Parallel columns

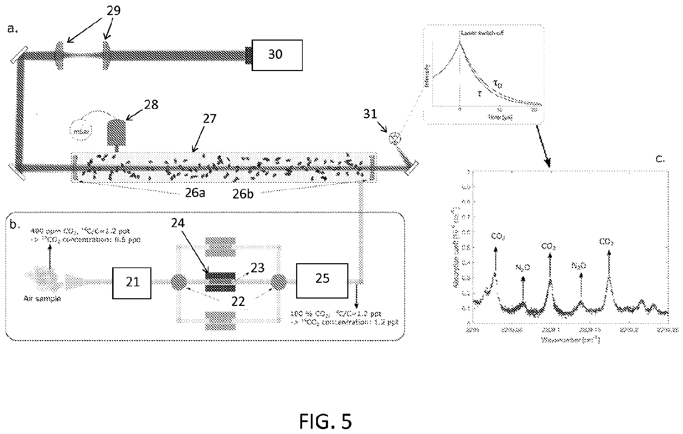

FIGURE 5 illustrates an experimental set-up for fast sampling in accordance with an embodiment of the present invention. The sample is filtrated by a particle filter 21 before leading the sample into anion exchange columns. Three parallel columns 23 are used to enable faster sampling. Multiport valves 22 are controlled so that the filtered air sample gas flow is directed to only one of the three columns at a time. Heaters 24 are placed around the columns for carrying out release of a concentrated CO₂ sample. In this embodiment, after extraction, any remaining N₂O is removed from the concentrated sample by catalytic conversion 25 by using a NiO/NaOH catalyst, to further improve the accuracy of the CO₂ determination. Cryogenic trapping is not needed. Thereafter, the sample is lead to a photometric measurement cell 27 comprising high-reflectivity mirrors 26a, 26b at both ends and a pressure sensor 28, and a laser spectroscopic measurement is carried out. The measurement set-up comprises a quantum cascade laser 30 as the light source, mode matching optics 29, and a photovoltaic detector 31. Section "c." shows the result of the measurement.

It should be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. **In** addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

In the following description, numerous specific details are provided, such as examples of lengths, widths, shapes, etc., to provide a thorough understanding of embodiments of the invention. In other instances, well-known structures, materials, or operations are not shown or described in detail to avoid obscuring aspects of the invention.

Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### INDUSTRIAL APPLICABILITY

The present invention is industrially applicable at least in the monitoring of radiocarbon gaseous emissions in the form of carbon dioxide and methane from atmospheric samples, typically emitted from nuclear power plants or radioactive waste repositories.

### REFERENCE SIGNS LIST

- 11: tunable light source
- 12: gas cell
- 13: detector
- 21: particle filter
- 22: multiport valves
- 23: resin
- 24: heater
- 25: catalytic conversion; N₂O removal
- 26a, 26b: high-reflectivity mirrors
- 27: measurement cell
- 28: pressure sensor
- 29: mode matching optics
- 30: quantum cascade laser
- 31: photovoltaic detector

### CITATION LIST

### Patent Literature

US 2007/0217982 A1
US 2017/284993 A1
Non Patent Literature
A. Yoshida et al., "Adsorption of CO2 on composites of strong and weak basic anion exchange resin and chitosan", J. Chem. Eng. of Japan 35 (2002) 32-39.
Fouladvand et al., "Methane Oxidation Over Pd Supported on Ceria-Alumina Under Rich/Lean Cycling Conditions", Topics in Catal. (2013) 56:410-415.
G. Genoud et al., "Radiocarbon dioxide detection based on cavity ring-down spectroscopy and a quantum cascade laser", Optics Letters 40 (2015) 1342-1345.
W. R. Alesi et al., "Evaluation of a Primary Amine-Functionalized Ion-Exchange Resin for CO2 Capture", Ind. Eng. Chem. Res. 51 (2012) 6907-6915.
I. Galli et al., "Spectroscopic detection of radiocarbon dioxide at parts-per-quadrillion sensitivity", Optica 3 (2016) 385-388.
A. J. Fleisher, D. A. Long, Q. Liu, L. Gameson, and J. T. Hodges, "Optical measurement of radiocarbon below unity fraction modern by linear absorption spectroscopy", The Journal of Physical Chemistry Letters 0, PMID: 28880564, 4550 (2017).
A. D. McCartt, T. Ognibene, G. Bench, and K. Turteltaub, "Measurements of carbon-14 with cavity ring-down spectroscopy", Nucl. Instr. Meth. Phys. Res. B 361, 277 (2015).

## Claims

1. A method of detecting carbon dioxide in a gaseous sample, the method comprising:
- flowing the gaseous sample through an anion exchange resin that is capable of selectively adsorbing CO₂ present in the gaseous sample;
- releasing the adsorbed CO₂ from the resin by heating the resin to a temperature in the range 80 to 250 °C to obtain a concentrated gaseous sample;
- determining the amount of an isotopic form of CO₂ in the concentrated gaseous sample by infrared absorption spectroscopy,
wherein the anion exchange resin features primary, secondary, and/or tertiary amino groups.

2. The method according to claim 1, wherein the isotopic form is ¹²CO₂ or ¹³CO₂ or ¹⁴CO₂ or any combination of them.

3. The method according to any of the preceding claims, wherein the anion exchange resin comprises crosslinked polymeric material.

4. The method according to any of the preceding claims, wherein the resin is heated to a temperature in the range 100 to 200 °C, for example 150 to 200 °C, and
wherein the resin is heated for a time period of 1 to 15 minutes, preferably for 10 minutes at maximum.

5. The method according to any of the preceding claims, wherein the determining step comprises measuring an infrared absorption spectrum of the concentrated gaseous sample by using a cavity ring-down laser spectroscopy.

6. The method according to any of the preceding claims, wherein:
the isotopic form is ¹⁴CO₂;
the gaseous sample further comprises ¹⁴CH₄, and the method further comprises, before the determination step:
- catalytically oxidizing the ¹⁴CH₄ to ¹⁴CO₂ by a catalyst,
whereby the ¹⁴CO₂ to be determined in the determination step also comprises ¹⁴CO₂ converted from the ¹⁴CH₄ present in the gaseous sample.

7. The method according to claim 6, wherein the catalyst is a Pd catalyst, and the step of catalytically oxidizing the ¹⁴CH₄ to ¹⁴CO₂ comprises:
- heating the Pd catalyst to a temperature of at least 300 °C;
- bringing the gaseous sample into contact with the heated Pd catalyst;
- whereby the heated Pd catalyst catalyses oxidation of the ¹⁴CH₄ present in the gaseous sample to ¹⁴CO₂.

8. The method according to any of the preceding claims, wherein
the isotopic form is ¹⁴CO₂ and the gaseous sample originates from a nuclear power plant, or
the gaseous sample is an atmospheric sample, or
the gaseous sample is/originates from biofuels, such as biodiesel or biogas, or
the gaseous sample is/originates from a biological sample, such as a breath sample, a blood sample or a plasma sample.

9. An apparatus comprising in a cascade:
- first means for concentrating CO₂ present in a gaseous sample to obtain a concentrated gaseous sample; and
- second means for determining the amount of an isotopic form of CO₂ present in the concentrated gaseous sample by infrared absorption spectroscopy,
wherein the first means comprises an anion exchange resin that is capable of selectively adsorbing CO₂ present in the gaseous sample,
wherein the anion exchange resin features primary, secondary, and/or tertiary amino groups.

10. The apparatus according to claim 9, wherein:
the isotopic form is ¹⁴CO₂; and
the apparatus further comprises:
- upstream of said first means, further means for catalytically oxidizing ¹⁴CH₄ present in the gaseous sample,
wherein said second means is adapted for determining the combined amount of ¹⁴CO₂ present in the gaseous sample and ¹⁴CO₂ converted from the ¹⁴CH₄ present in the gaseous sample by infrared absorption spectroscopy,
wherein preferably the further means for catalytically oxidizing ¹⁴CH₄ present in the gaseous sample comprises a catalyst bed comprising a catalyst, preferably a Pd catalyst.

11. The apparatus according to any of claims 9 to 10, wherein the second means comprises a cavity ring-down laser spectrometer comprising a quantum cascade laser as an IR light source.

## Patentansprüche

1. Verfahren zum Detektieren von Kohlendioxid in einer gasförmigen Probe, wobei das Verfahren Folgendes umfasst:
- Strömen der gasförmigen Probe durch ein Anionenaustauschharz, das fähig ist, das in der gasförmigen Probe vorhandene CO₂ selektiv zu adsorbieren;
- Freisetzen des adsorbierten CO₂ aus dem Harz durch Erhitzen des Harzes auf eine Temperatur im Bereich von 80 bis 250 °C, um eine konzentrierte gasförmige Probe zu erhalten;
- Bestimmen der Menge einer Isotopenform von CO₂ in der konzentrierten gasförmigen Probe durch Infrarot-Absorptionsspektroskopie,
wobei das Anionenaustauschharz primäre, sekundäre und/oder tertiäre Aminogruppen aufweist.

2. Verfahren nach Anspruch 1, wobei die Isotopenform ¹²CO₂ oder ¹³CO₂ oder ¹⁴CO₂ oder eine beliebige Kombination davon ist.

3. Verfahren nach einem der vorstehenden Ansprüche, wobei das Anionenaustauschharz ein vernetztes Polymermaterial umfasst.

4. Verfahren nach einem der vorstehenden Ansprüche, wobei das Harz auf eine Temperatur im Bereich von 100 bis 200 °C, beispielsweise 150 bis 200 °C, erhitzt wird, und
wobei das Harz für einen Zeitraum von 1 bis 15 Minuten, bevorzugt höchstens 10 Minuten erhitzt wird.

5. Verfahren nach einem der vorstehenden Ansprüche, wobei der Bestimmungsschritt Messen eines Infrarot-Absorptionsspektrums der konzentrierten gasförmigen Probe durch Verwenden von Cavity-Ring-Down-Laserspektroskopie umfasst.

6. Verfahren nach einem der vorstehenden Ansprüche, wobei:
die Isotopenform ¹⁴CO₂ ist;
die gasförmige Probe weiter ¹⁴CH₄ umfasst und das Verfahren vor dem Bestimmungsschritt weiter Folgendes umfasst:
- katalytisches Oxidieren von ¹⁴CH₄ zu ¹⁴CO₂ durch einen Katalysator,
wobei das im Bestimmungsschritt zu bestimmende ¹⁴CO₂ auch ¹⁴CO₂ umfasst, das aus dem in der gasförmige Probe vorhandenen ¹⁴CH₄ umgewandelt wurde.

7. Verfahren nach Anspruch 6, wobei der Katalysator ein Pd-Katalysator ist und der Schritt des katalytischen Oxidierens von ¹⁴CH₄ zu ¹⁴CO₂ Folgendes umfasst:
- Erhitzen des Pd-Katalysators auf eine Temperatur von mindestens 300 °C;
- Herstellen von Kontakt der gasförmigen Probe mit dem erhitzten Pd-Katalysator;
- wobei der erhitzte Pd-Katalysator Oxidation des in der gasförmigen Probe vorhandenen ¹⁴CH₄ zu ¹⁴CO₂ katalysiert.

8. Verfahren nach einem der vorstehenden Ansprüche, wobei
die Isotopenform 14CO2 ist und die gasförmige Probe aus einem Kernkraftwerk stammt
oder
die gasförmige Probe eine atmosphärische Probe ist oder
die gasförmige Probe aus Biokraftstoffen, wie Biodiesel oder Biogas ist oder stammt oder
die gasförmige Probe aus eine biologischen Probe, wie einer Atemprobe, einer Blutprobe oder einer Plasmaprobe ist oder stammt.

9. Einrichtung, umfassend in einer Kaskade:
- erste Mittel zum Konzentrieren von in einer gasförmigen Probe enthaltenem CO₂, um eine konzentrierte gasförmige Probe zu erhalten; und
- zweite Mittel zum Bestimmen der Menge einer Isotopenform von CO2, die in der konzentrierten gasförmige Probe vorhanden ist, durch Infrarot-Absorptionsspektroskopie,
wobei die ersten Mittel ein Anionenaustauschharz umfassen, das fähig ist, das in der gasförmigen Probe vorhandene CO₂ selektiv zu adsorbieren, wobei das Anionenaustauschharz primäre, sekundäre und/oder tertiäre Aminogruppen aufweist.

10. Einrichtung nach Anspruch 9, wobei:
die Isotopenform ¹⁴CO₂ ist; und
die Einrichtung weiter Folgendes umfasst:
- vorgelagert vor den ersten Mitteln, weitere Mittel zum katalytischen Oxidieren des in der gasförmigen Probe vorhandenen ¹⁴CH₄,
wobei die zweiten Mittel so angepasst sind, dass sie die kombinierte Menge von ¹⁴CO₂, die in der gasförmigen Probe vorhanden ist, und aus dem ¹⁴CH₄ umgewandeltes ¹⁴CO₂, das in der gasförmigen Probe vorhanden ist, durch Infrarot-Absorptionsspektroskopie bestimmen,
wobei die weiteren Mittel zum katalytischen Oxidieren von in der gasförmigen Probe vorhandenem ¹⁴CH₄ bevorzugt ein Katalysatorbett umfassen, das einen Katalysator, bevorzugt einen Pd-Katalysator, umfasst.

11. Einrichtung nach einem der Ansprüche 9 bis 10, wobei die zweiten Mittel ein Cavity-Ring-Down-Laserspektrometer umfassen, das einen Quantenkaskadenlaser als IR-Lichtquelle umfasst.

## Revendications

1. Procédé de détection de dioxyde de carbone dans un échantillon gazeux, le procédé comprenant :
- le passage de l'échantillon gazeux à travers une résine échangeuse d'anions capable d'adsorber sélectivement le CO₂ présent dans l'échantillon gazeux ;
- la libération du CO₂ adsorbé à partir de la résine en chauffant la résine à une température dans la plage allant de 80 à 250 °C pour obtenir un échantillon gazeux concentré ;
- la détermination de la quantité d'une forme isotopique de CO₂ dans l'échantillon gazeux concentré par spectroscopie d'absorption infrarouge,
dans lequel la résine échangeuse d'anions est **caractérisée par** des groupes amino primaires, secondaires, et/ou tertiaires.

2. Procédé selon la revendication 1, dans lequel la forme isotopique est ¹²CO₂ ou ¹³CO₂ ou ¹⁴CO₂, ou toute combinaison de celles-ci.

3. Procédé selon l'une quelconque des revendications précédentes, dans lequel la résine échangeuse d'anions comprend un matériau polymère réticulé.

4. Procédé selon l'une quelconque des revendications précédentes, dans lequel la résine est chauffée à une température dans la plage allant de 100 à 200 °C, par exemple de 150 à 200 °C, et
dans lequel la résine est chauffée pendant une durée de 1 à 15 minutes, de préférence pendant 10 minutes au maximum.

5. Procédé selon l'une quelconque des revendications précédentes, dans lequel l'étape de détermination comprend la mesure d'un spectre d'absorption infrarouge de l'échantillon gazeux concentré à l'aide d'une spectroscopie laser à cavité optique.

6. Procédé selon l'une quelconque des revendications précédentes, dans lequel :
la forme isotopique est ¹⁴CO₂ ;
l'échantillon gazeux comprend en outre ¹⁴CH₄, et le procédé comprend en outre, avant l'étape de détermination :
- l'oxydation catalytique du ¹⁴CH₄ en ¹⁴CO₂ par l'intermédiaire d'un catalyseur,
selon lequel le ¹⁴CO₂ à déterminer dans l'étape de détermination comprend également le ¹⁴CO₂ converti à partir du ¹⁴CH₄ présent dans l'échantillon gazeux.

7. Procédé selon la revendication 6, dans lequel le catalyseur est un catalyseur au Pd, et l'étape d'oxydation catalytique du ¹⁴CH₄ en ¹⁴CO₂ comprend :
- le chauffage du catalyseur au Pd à une température d'au moins 300 °C ;
- la mise en contact de l'échantillon gazeux avec le catalyseur au Pd chauffé ;
- selon lequel le catalyseur au Pd chauffé catalyse l'oxydation du ¹⁴CH₄ présent dans l'échantillon gazeux en ¹⁴CO₂.

8. Procédé selon l'une quelconque des revendications précédentes, dans lequel
la forme isotopique est 14CO2 et l'échantillon gazeux provient d'une centrale nucléaire,
ou
l'échantillon gazeux est un échantillon atmosphérique, ou
l'échantillon gazeux est/provient de biocarburants, tels que le biodiesel ou le biogaz, ou
l'échantillon gazeux est/provient d'un échantillon biologique, tel qu'un échantillon d'haleine, un échantillon de sang ou un échantillon de plasma.

9. Appareil, comprenant en cascade :
- un premier moyen pour concentrer le CO₂ présent dans un échantillon gazeux afin d'obtenir un échantillon gazeux concentré ; et
- un second moyen pour déterminer la quantité d'une forme isotopique de CO2 présente dans l'échantillon gazeux concentré par spectroscopie d'absorption infrarouge,
dans lequel le premier moyen comprend une résine échangeuse d'anions capable d'adsorber sélectivement le CO₂ présent dans l'échantillon gazeux, dans lequel la résine échangeuse d'anions se **caractérise par** des groupes amino primaires, secondaires et/ou tertiaires.

10. Appareil selon la revendication 9, dans lequel
la forme isotopique est ¹⁴CO₂ ; et
l'appareil comprend en outre :
- en amont dudit premier moyen, un moyen supplémentaire pour une oxydation catalytique du ¹⁴CH₄ présent dans l'échantillon gazeux,
dans lequel ledit second moyen est conçu pour déterminer la quantité combinée de ¹⁴CO₂ présente dans l'échantillon gazeux et de ¹⁴CO₂ converti à partir du ¹⁴CH₄ présent dans l'échantillon gazeux par spectroscopie d'absorption infrarouge,
dans lequel, de préférence, le moyen supplémentaire d'oxydation catalytique du ¹⁴CH₄ présent dans l'échantillon gazeux comprend un lit catalytique comprenant un catalyseur, de préférence un catalyseur au Pd.

11. Appareil selon l'une quelconque des revendications 9 à 10, dans lequel le second moyen comprend un spectromètre laser à cavité optique comprenant un laser à cascade quantique comme source de lumière IR.
